# FASCICULE DE BREVET EUROPEEN

(11) **EP 1 044 673 B1**
(45) Date de publication et mention de la délivrance du brevet: **11.07.2001**
(21) Numéro de dépôt: 00400916.3
(22) Date de dépôt: 03.04.2000
(51) Int. Cl.: A61K 7/02

(54) **Composition cosmétique sous forme anhydre comprenant une dispersion de particules de polymère stabilisées en surface**
Wasserfreie kosmetische Zusammensetzung bestehend aus einer Dispersion von Oberflächenstabilisierten Polymerteilchen
Anhydrous cosmetic composition containing a dispersion of surface stabilized polymer particles

(30) Priorité: 16.04.1999 FR 9904815
(43) Date de publication de la demande: 18.10.2000
(73) Titulaire: L'OREAL, 75008 Paris (FR)
(72) Inventeur: Chevalier, Véronique, 94440 Villecresnes (FR); Hurel, Valérie, 91190 Gif sur Yvette (FR)
(74) Mandataire: Lhoste, Catherine

(56) Documents cités:
- EP-A- 0 400 546
- EP-A- 0 504 066
- EP-A- 0 797 976
- WO-A-94/13628
- WO-A-97/01321

## Description

La présente invention a trait à une composition sous forme anhydre pour le soin et/ou le maquillage de la peau aussi bien du visage que du corps humain, contenant un polymère dispersible dans une phase grasse et une matière colorante. Cette composition destinée aux domaines cosmétique et dermatologique permet notamment le camouflage des taches (vitiligo), des défauts de pigmentation de la peau, des dischromies (angiômes) et de la couperose. Cette composition présente, en outre, des propriétés remarquables de résistance à l'eau et de non transfert.

Cette composition peut se présenter notamment sous forme de pâte, de gel ou de crème plus ou moins fluide et être un fond de teint, une composition teintée de soin de la peau, de protection solaire, de bronzage artificiel de la peau ou de maquillage du corps ou encore un fard à paupières ou à joues, un produit anti-cerne.

Pour camoufler les taches, notamment de vieillesse, dûes à une mélanogénèse altérée, les défauts de pigmentation de la peau, les dischromies, la couperose, les irrigations diffuses (apparitions de petits vaisseaux sanguins sous la peau) et les télangiectasies, on utilise généralement des fonds de teint à pouvoir couvrant élevé. Ces fonds de teint contiennent généralement des phases grasses telles que des cires et des huiles, des pigments et/ou charges et, éventuellement des additifs comme des actifs cosmétiques ou dermatologiques. Ils peuvent aussi contenir des produits dits "pâteux", de consistance souple, permettant d'obtenir des pâtes colorées à appliquer au pinceau, au doigt ou à l'éponge.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact, et notamment un vêtement ou la peau. Il s'ensuit une persistance médiocre du film appliqué, nécessitant de renouveler régulièrement l'application de la composition de fond de teint. Par ailleurs, l'apparition de ces traces inacceptables notamment sur les cols de chemisier écarte certaines femmes de l'utilisation de ce type de produit.

Dans le document EP-A-709083, il est décrit des fonds de teint contenant des silicones volatiles et une résine de silicone à structure tridimensionnel, présentant des propriétés de "sans transfert" . Malheureusement, ces fonds de teint améliorés ont l'inconvénient de laisser sur la peau, après évaporation des huiles de silicone, un film qui devient inconfortable au cours du temps (sensation de dessèchement et de tiraillement), écartant encore certaines femmes de ce type de produits. Pour améliorer le confort de ces produits, on pourrait leur ajouter des huiles non volatiles siliconées ou non, mais dans ce cas particulier, on perdrait en efficacité "sans transfert".

Plus récemment, on a envisagé dans le document EP-A-775483 des compositions pour la peau sous forme de dispersion aqueuse de polymère filmogène. Ces compositions présentent de bonnes propriétés de "sans transfert" et de rémanence à l'eau. Malheureusement, ces films présentent l'inconvénient d'être inconfortables au cours du temps, après évaporation de l'eau.

Par ailleurs, il n'existe pas à ce jour de moyen simple et durable dans le temps pour cacher les taches des mains et du corps des êtres humains, ne tachant pas les vêtements, résistant à l'eau, à la sueur et au sébum.

Il subsiste donc le besoin d'une composition ne présentant pas les inconvénients ci-dessus, et ayant notamment des propriétés de camouflage des taches et autres dischromies de la peau, irrigations sanguines diffuses, durable dans le temps, tout en ayant des propriétés de "sans transfert" remarquables, même lors d'une pression ou d'un frottement prononcé, ne desséchant pas la peau sur laquelle elle est appliquée, aussi bien lors de l'application qu'au cours du temps.

La demanderesse a constaté, de façon tout à fait surprenante, que l'utilisation d'une phase grasse liquide particulière dans laquelle sont dispersées et stabilisées des particules de polymère permettait d'obtenir une composition cosmétique ou dermatologique, conduisant à un dépôt cohésif couvrant et camouflant, de très bonne tenue, ne transférant pas du tout, résistant à l'eau, à la sueur et au sébum, tout en étant très agréable à l'application et à porter tout au long de la journée. Le dépôt est notamment ni gras, ni sec, flexible et non collant.

La présente invention a donc pour objet une composition susceptible d'être appliquée sur la peau se présentant sous forme anhydre, comprenant une phase grasse liquide, des particules de polymère dispersées et stabilisées en surface dans ladite phase grasse liquide et une matière colorante, la phase grasse liquide contenant une phase liquide volatile et une phase liquide non volatile, la phase non volatile ayant un paramètre de solubilité δₕ ≤ 5 (J/cm³)^{1/2} et le rapport, en poids, phase volatile/phase non volatile allant de 2 à 25.

Par "phase grasse liquide", on entend tout milieu non aqueux liquide à température ambiante (25°C) et pression atmosphérique (760 mm de Hg). Par "phase liquide volatile", on entend tout milieu non aqueux susceptible de s'évaporer au contact de la peau en moins d'une heure, à température ambiante et pression atmosphérique. Cette phase volatile comporte notamment des huiles ayant une pression de vapeur non nulle, à température ambiante et pression atmosphérique, allant en particulier de 10⁻³ à 300mm de Hg (0,13 Pa à 40 000 Pa). Les huiles ne satisfaisant pas à cette condition doivent être considérées comme des huiles non volatiles.

Pour un rapport en poids de phase volatile/phase non volatile, rapport noté R, inférieur à 2, on obtient sur la peau un film qui ne sèche pas, qui est poisseux et qui transfère. Le camouflage n'est donc pas rémanent. Pour un rapport R supérieur à 25, on obtient un film qui sèche beaucoup trop vite conduisant à la formation d'une poudre qui n'adhère pas sur la peau. De préférence R est choisi dans la gamme de 5 à 15.

Cette composition est en particulier une composition cosmétique ou dermatologique. Elle contient donc des ingrédients compatibles avec la peau.

Cette composition, en plus, des avantages mentionnés précédemment, peut être transparente (à savoir procurer un maquillage naturel), capable d'homogénéiser la couleur de la peau instantanément et de façon durable (un jour environ, même en se lavant). Le film obtenu présente, en outre, l'avantage de se démaquiller de façon aisée avec un démaquillant classique (lotion ou lait démaquillant). Par ailleurs, ces compositions sont très stables : aucune décantation et/ou déphasage au bout de 2 mois à 45°C et au bout de 3 ans à température ambiante.

La composition de l'invention est parfaitement bien adaptée au soin et à la protection des mains.

La quantité de polymère doit être suffisante pour former sur la peau du visage et/ou du corps, y compris le cou, les pieds et les mains, un film apte à piéger les matières colorantes en vue de limiter, voire supprimer, leur transfert sur un support avec lequel le film est mis en contact. La quantité de polymère est notamment fonction de la quantité de matières colorantes contenue dans la composition. En pratique, la quantité de polymère est supérieure à 2 % en poids (en matière active), par rapport au poids total de la composition.

Un autre objet de l'invention est l'utilisation dans une composition cosmétique ou pour la fabrication d'une composition physiologiquement acceptable de particules de polymère dispersées et stabilisées en surface dans une phase grasse liquide, pour diminuer, voire supprimer les taches, les dischromies, les défauts de pigmentation, la couperose et/ou les irrigations sanguines diffuses apparaissant à travers la peau, ladite phase grasse liquide contenant une phase liquide volatile et une phase liquide non volatile, la phase non volatile ayant un paramètre de solubilité δₕ ≤ 5 (J/cm³)^{1/2} et le rapport, en poids, phase volatile/phase non volatile allant de 2 à 25, ladite composition contenant une matière colorante.

L'invention a aussi pour objet un procédé de soin cosmétique ou de maquillage de la peau, consistant à appliquer sur la peau une composition cosmétique telle définie précédemment.

L'invention a encore pour objet un procédé non thérapeutique (en particulier cosmétique) de camouflage notamment rémanent des taches, des défauts de pigmentation de la peau, des dischromies, de la couperose, des irrigations sanguines diffuses apparaissant à travers la peau et des télangiectasies, consistant à introduire dans une composition contenant une matière colorante, une phase grasse liquide contenant une phase liquide volatile, une phase liquide non volatile et des particules de polymère dispersées et stabilisées en surface dans ladite phase grasse liquide, la phase non volatile ayant un paramètre de solubilité δₕ ≤ 5 (J/cm³)^{1/2} et le rapport, en poids, phase volatile/phase non volatile allant de 2 à 25.

Avantageusement, la composition contient au moins un ingrédient choisi parmi les actifs cosmétiques, dermatologiques et leurs mélanges. Grâce à la dispersion de particules de polymère stabilisées en surface présente dans la phase grasse liquide, la composition de l'invention permet de limiter, voire supprimer le transfert de la composition et en particulier le transfert des matières colorantes et donc de maintenir ces matières colorantes là où elles ont été déposées, de résister à l'eau, au sébum et à la sueur.

### Polymère en dispersion

Selon l'invention le polymère est insoluble dans la phase grasse même à sa température de ramollissement, à l'inverse d'une cire même d'origine polymérique qui est elle soluble dans la phase grasse à sa température de fusion. Il permet, en outre, la formation d'un dépôt filmogène se présentant sous forme de film isolable, continu et homogène et/ou est caractérisé par l'enchevêtrement des chaînes polymériques. Avec une cire même obtenue par polymérisation on obtient, après fusion dans la phase grasse, une recristallisation.

Le polymère utilisé dans la présente demande peut être de toute nature. On peut ainsi employer un polymère radicalaire, un polycondensat, voire un polymère d'origine naturelle et leurs mélanges. Le polymère peut être choisi par l'homme du métier en fonction de ses propriétés et selon l'application ultérieure souhaitée pour la composition. De préférence le polymère utilisé est non filmifiable.

Par polymère non filmifiable, on entend un polymère non capable de former, seul, un film isolable. Ce polymère permet, en association avec la phase non volatile, de former un dépôt continue et homogène sur la peau.

De façon avantageuse, le polymère se présente sous forme de particules dispersées et stabilisées en surface par au moins un stabilisant.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau. Ceci ne serait pas possible avec des pigments sous forme particulaire, leur constitution ne permettant pas de moduler la taille moyenne des particules.

On a de plus constaté que les compositions selon l'invention, présentent des qualités d'étalement et d'adhésion sur la peau particulièrement intéressantes, ainsi qu'un toucher onctueux et agréable. Ces compositions ont, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte supplémentaire pour l'utilisatrice.

Les compositions selon l'invention comprennent donc avantageusement une dispersion stable de particules généralement sphériques d'au moins un polymère, dans une phase grasse liquide physiologiquement acceptable. Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm, les dispersions de particules deviennent beaucoup moins stables.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère mou à un polymère plus ou moins dur, permettant de régler les propriétés mécaniques de la compositions en fonction de l'application envisagée. La modification de la température de transition vitreuse peut être réalisée notamment avec un des plastifiants usuellement utilisés dans les domaines d'application concernés et notamment parmi les composés susceptibles d'être des solvants du polymère.

Les polymères utilisables dans la composition de l'invention ont de préférence un poids moléculaire en nombre de l'ordre de 2000 à 10 000 000 et une (Tg) de -100°C à 300°C et mieux de - 10° à 50°C.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à la température de la peau et notamment inférieure ou égale à 40°C et notamment allant de - 10° à 30°C.

Parmi les polymères non filmifiables utilisables dans l'invention, on peut citer des homopolymères ou copolymères radicalaires, vinyliques ou acryliques, éventuellement réticulés, ayant de préférence une Tg supérieure ou égale à 40°C et notamment allant de 45 à 150°C.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tes que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en C₁-C₂₀, de préférence en C₁-C₈, les (méth)acrylates d'aryle, en particulier d'aryle en C₆-C₁₀, les (méth)acrylates d'hydroxyalkyle, en particulier d'hydroxyalkyle en C₂-C₆. Comme (méth)acrylates d'alkyle, on peut citer le (méth)acrylate de méthyle, d'éthyle, de butyle, d'isobutyle, d'éthyl-2 hexyle et le lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymère d'acide (méth)acrylique et de (méth)acrylate d'alkyle, notamment d'alkyle en C₁-C₄. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth)acrylamides, en particulier d'alkyl en C₂-C₁₂ tels que le N-éthyl acrylamide, le N-t-butyl acrylamide, le N-octyl acrylamide ; les N- dialkyl (C₁-C₄) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyl, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :
- la N-vinylpyrrolidone, la vinylcaprolactame, les vinyl N-alkyl(C₁-C₆) pyrroles, les vinyloxazoles; les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide de monomère difonctionnel, notamment comprenant au moins deux insaturations éthyléniques, tel que le diméthacrylate d'éthylène glycol ou le phtalate de diallyle.

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

### Phase grasse liquide

La phase grasse liquide dans laquelle est dispersé le polymère, peut être constituée de toute huile cosmétiquement ou dermatologiquement acceptable, et de façon plus générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée, l'une de ses huiles au moins ayant un paramètre de solubilité δₕ ≤ 5. Cette huile ou ces huiles de paramètre de solubilité δₕ ≤ 5, qui seront appelées huile primaires, sont compatibles avec la matière colorante et les particules de polymère, c'est-à-dire qu'elle évitent notamment toute ségrégation de ces particules de polymère et de cette matière colorante. Ces huiles primaires permettent, en outre, une solubilisation des huiles de paramètre de solubilité δₕ > 5, appelées huiles secondaires, rendant ainsi ces huiles compatibles avec les particules de polymère et la matière colorante.

La définition des corps gras dans l'espace de solubilité tridimensionnel selon HANSEN est décrite dans l'article de C. M. HANSEN : "The three dimensionnal solubility parameters" J. Paint Technol. 39, 105 (1967) et dans l'article "Solubility parameter values" de Eric A. Grulke de l'ouvrage "Polymer Handbook" 3^{ème} édition, Chapitre VII, pages 519-559.

Selon cet espace de Hansen : δₕ caractérise les forces d'interactions spécifiques (type liaisons hydrogène, acide/base, donneur/accepteur, etc...).

Comme huile primaire non volatile utilisable dans l'invention, on peut citer les huiles apolaires ou peu polaires, c'est-à-dire les huiles comportant une chaîne alkyle notamment en C₃-C₄₀. Comme exemple d'huile primaire, on peut citer :
- les hydrocarbures linéaires ou ramifiés tels que les huiles de paraffine, de vaseline et de naphtalène légères, la lanoline ;
- les huiles hydrocarbonées d'origine animale comme le squalène ;
- les huiles hydrocarbonées végétales telles que les triglycérides liquides d'acides gras de 4 à 10 atomes de carbone comme les huiles de tournesol, d'olive, de bourrache, de maïs, de son de riz, de germes de blé, de soja, de courge, de rosier muscat, de pépins de cassis, de sésame, de noisette, d'abricot, d'arara, de macadamia, d'avocat, de jojoba, de limnanthes alba raffinée ;
- les esters et les éthers de synthèse notamment d'acides gras comme les huiles de formule R₁CO(O)ₓR₂ dans laquelle R₁ représente le reste d'un acide comportant de 2 à 29 atomes de carbone avec x valant 0 ou 1 et R₂ représente une chaîne hydrocarbonée contenant de 3 à 30 atomes de carbone comme par exemple l'acétyl-citrate de tributyle, l'érucate d'oléyle, le béhénate d'octyl-2 dodécyle, le citrate de tri-iso-arachidyle, le stéaroyl-stéarate d'isocétyle ou d'octyldodécanyle, l'acétate de n-propyle, le tri-méllitate de tridécyle, le dodécane di-oléate ou le stéarate de di-iso-cétyle, le propionate d'arachidyle, le dibutyl phtalate, le carbonate de propylène, le pentanoate d'octyldodécyle ; les esters de polyol comme la vitamine F, l'isostéarate de sorbitane, le triisostéarate de glycérine ou de diglycérine ;
- les huiles siliconées telles que les polydiméthylsiloxanes (PDMS), comportant éventuellement une chaîne alkyle ou alcoxy en C₃-C₄₀ ou une chaîne phénylée telle que les phényltriméthicones, les polyalkylméthylsiloxanes, éventuellement fluorés comme les polyméthyltrifluoro-propyldiméthylsiloxanes, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ;
- leurs mélanges.

Ces huiles primaires non volatiles peuvent représenter de 1 à 15 % du poids total de la composition et mieux de 5 à 10 %.

A ces huiles primaires, on peut éventuellement ajouter une ou plusieurs huiles secondaires choisies parmi les hydrocarbures linéaires ou ramifiés comme le polyisobutylène hydrogéné ; les esters d'acide gras de 7 à 29 atomes de carbone comme le malate de diisostéaryle, le palmitate d'isopropyle, l'adipate de diisopropyle, les triglycérides des acides caprylique/caprique comme ceux vendus par la société Stearineries Dubois ou ceux vendus sous les dénominations Miglyol 810, 812 et 818 par la société Dynamit Nobel, l'huile de beurre de karité, le myristate d'isopropyle, le stéarate de butyle, le laurate d'hexyle, l'adipate de diisopropyle, l'isononate d'isononyle, le laurate de 2-hexyl-décyle, le palmitate de 2-octyl-décyle, le myristate ou le lactate de 2-octyl-dodécyle, le succinate de 2-diéthyl-hexyle, le palmitate d'éthyl-2hexyle, le stéarate d'octyl-2-dodécyle, l'huile de ricin ; les esters d'acide lanolique, d'acide laurique, d'acide stéarique ; les alcools gras supérieurs (de 7 à 29 atomes de carbone) tels que l'alcool stéarylique, l'alcool linoléique ou linolénique, l'alcool isostéarique, l'octyl 2-dodécanol, le décanol, le dodécanol, l'octadécanol ou l'alcool oléique ; les acides gras supérieurs (de 7 à 29 atomes de carbone) tels que l'acide myristique, l'acide palmitique, l'acide stéarique, l'acide béhénique, l'acide oléique, l'acide linoléique, l'acide linolénique ou l'acide isostéarique ; leurs mélanges.

Ces huiles secondaires peuvent représenter de 0 à 10 % du poids total de la composition et mieux de 0 à 5 %.

Selon l'invention, on peut, en outre, utiliser une ou plusieurs huiles volatiles à température ambiante et pression atmosphérique. Ces huiles volatiles facilitent, notamment, l'application de la composition sur la peau. Ces huiles peuvent être des huiles hydrocarbonées, des huiles siliconées comportant éventuellement des groupements alkyle ou alkoxy pendants ou en bout de chaîne siliconée, des esters d'acide et d'alcool inférieurs (en C₁ à C₈).

Comme huile de silicone volatile utilisable dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme autre huile volatile utilisable dans l'invention, on peut citer notamment les huiles isoalcanes (appelées aussi isoparaffines) en C₈-C₁₆ comme l'isododécane, l'isodécane, l'isohexadécane et par exemple les huiles vendus sous les noms commerciaux d'ISOPARs', de PERMETYLs et notamment l'isododécane (PERMETYLs 99 A), les esters ramifiés en C₈-C₁₆ comme le néo pentanoate d'iso-hexyle, et leurs mélanges.

De préférence, on utilise comme huile volatile une ou plusieurs huiles ayant un paramètre de solubilité δₕ ≤ 5 et notamment l'isododécane et les isoparaffines en C₈-C₁₆.

Ces huiles volatiles représentent notamment de 5 à 85 % du poids total de la composition, et mieux de 20 à 75 %.

Le choix du milieu non aqueux est effectué par l'homme du métier en fonction de la nature des monomères constituant le polymère et/ou de la nature du stabilisant, comme indiqué ci-après.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie contient une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

### Stabilisant

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly-12-(hydroxystéarique).

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins un polyéther. Le bloc polyorganopolysiloxane peut être notamment un polydiméthylsiloxane ou bien encore un poly alkyl(C₂-C₁₈) méthyl siloxane ; le bloc polyéther peut être un poly alkylène en C₂-C₁₈, en particulier polyoxyéthylène et/ou polyoxypropylène. En particulier, on peut utiliser les diméthicones copolyol ou des alkyl (C₂-C₁₈) diméthicones copolyol tels que ceux vendus sous la dénomination "DOW CORNING 3225C" par la société DOW CORNING, les lauryl méthicones tels que ceux vendu sous la dénomination "DOW CORNING Q2-5200 par la société **"DOW CORNING".**

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjugués comme l'éthylène ou les diènes tels que le butadiène et l'isoprène et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'KRATON' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile); OS129880, OS129881 et OS84383 de chez Lubrizol (copolymère styrène/méthacrylate).

Comme copolymères blocs greffés ou séquencés comprenant au moins un bloc résultant de la polymérisation d'un monomère à une ou plusieurs liaisons éthyléniques comme les diènes et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'un monomère à une ou plusieurs liaisons éthyléniques et d'au moins un bloc d'un polyéther tel qu'un polylkylène en C₂-C₁₈ (polyoxyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en C₁-C₄, et d'acrylates ou de méthacrylates d'alkyle issus d'alcools en C₈-C₃₀. On peut en particulier citer le copolymère méthacrylate de stéaryle/méthacrylate de méthyle.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

D'autre part, lorsque la phase grasse liquide comprend au moins une huile de silicone, l'agent stabilisant est de préférence choisi dans le groupe constitué par les copolymères blocs greffés ou séquencés comprenant au moins un bloc de type polyorganosiloxane résultant de la polymérisation d'un siloxane et au moins un bloc d'un polymère radicalaire ou d'un polyéther ou d'un polyester comme les blocs polyoxypropyléné et/ou oxyéthyléné.

Lorsque le phase grasse liquide ne comprend pas d'huile de silicone, l'agent stabilisant est de préférence choisi dans le groupe constitué par :
- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en C₁-C₄, et d'acrylates ou de méthacrylates d'alkyle issus d'alcools en C₈-C₃₀,
- (c) les copolymères blocs greffés ou séquencés comprenant au moins un bloc résultant de la polymérisation de monomère à un ou plusieurs liaisons éthyliniques comme les diènes,
et au moins un bloc d'un polymère vinylique ou acrylique ou d'un polyéther ou d'un polyester, ou leurs mélanges.

De préférence, on utilise des polymères dibloc comme agent stabilisant.

Les dispersions obtenues peuvent alors être utilisées dans une composition notamment cosmétique ou dermatologique telle qu'une composition de soin ou de maquillage de la peau à propriétés camoufflantes.

La composition comprend avantageusement une ou plusieurs matières colorantes contenant un ou plusieurs composés pulvérulents et/ou un ou plusieurs colorants liposolubles, par exemple à raison de 0,01 à 70% du poids total de la composition. Les composés pulvérulents peuvent être choisis parmi les pigments et/ou les nacres. De plus des charges telles que celles habituellement utilisées dans les domaines considérés peuvent être prévues.

Avantageusement, les composés pulvérulents représentent de 0,1 à 30 % du poids total de la composition de préférence de 1 à 20 % et mieux de 1 à 10%. Plus la quantité de composés pulvérulents diminue, plus les qualités de sans transfert et de confort augmentent. Le fait que les propriétés de sans transfert augmentent au fur et à mesure que la quantité de composés pulvérulents diminue est tout à fait surprenant. En effet, jusqu'à ce jour les propriétés de sans transfert des compositions de l'art antérieur augmentaient avec la quantité de composés pulvérulents. Inversement, leurs inconforts et leur sécheresse sur la peau ou muqueuses augmentaient.

Par ailleurs, la propriété de sans transfert augmente avec la quantité de polymère dispersible dans la phase grasse liquide. En pratique, le polymère peut représenter en matière active jusqu'à 30 % (en matière active ou sèche) du poids total de la composition. En utilisant au-dessus de 12 % en poids, de matière active de polymère et d'huile non volatile, dans la composition, on obtient un film sans transfert total. Entre 2 % et 12 % l'effet sans transfert est notable sans toutefois être total. On peut donc adapter les propriétés de sans transfert à volonté, ce qui n'était pas possible avec les compositions sans transfert de l'art antérieur, sans nuire au confort du film déposé.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0,01 à 20 % du poids de la compositions et mieux de 0,1 à 6 %.

Le polymère de la composition de l'invention permet la formation d'un film sur la peau formant un réseau, piégeant les matières colorantes (y compris les charges) et/ou les actifs. Selon la quantité relative de matières colorantes, utilisée par rapport à la quantité de polymère stabilisé, utilisée, il est possible d'obtenir un film plus ou moins brillant et plus ou moins sans transfert.

Comme actifs physiologiquement acceptables (notamment cosmétiques), on peut citer les vitamines, les acides gras essentiels, les sphingolipides, les filtres solaires, les actifs anti-radicaux libres (vitamine E notamment), les antipigmentants, les agents kératolytiques. Ces actifs sont utilisés en quantité habituelle pour l'homme du métier et notamment à des concentrations de 0,001 à 20 % du poids total de la composition.

La composition selon l'invention peut, de plus, comprendre, selon le type d'application envisagée, les constituants classiquement utilisés dans les domaines considérés, qui sont présents en une quantité appropriée à la forme galénique souhaitée.

En particulier, elle peut comprendre, outre, la phase grasse liquide dans laquelle le polymère est stabilisé des phases grasses additionnelles qui peuvent être choisies parmi les cires, les gommes et/ou les corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. Comme cire liquide à température ambiante, on peut citer l'huile de Jojoba. De préférence, on utilise des cires ayant un point de fusion supérieur à 45°C.
Les cires peuvent être présentes à raison de 0-30% en poids dans la composition et mieux de 0,5 à 10 %.

La composition peut comprendre, en outre, tout additif usuellement utilisé dans de telles compositions, tel que des épaississants, des antioxydants, des parfums, des conservateurs, des polymères liposolubles comme les polyalkylènes, notamment le polybutène, les polyacrylates et les polymères siliconés compatibles avec la phase grasse ainsi que les dérivés de polyvinylpyrolidone. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'une crème ou gel plus ou moins fluide, d'une pâte plus ou moins visqueuse, d'un gel anhydre solide coulé dans un moule en forme de coupelle ou de stick.

La composition de l'invention peut être un produit de maquillage de la peau comme les fonds de teint, les fard à joues ou à paupières, les produits de maquillage du corps (tatouage semi-permanent). Ces produits peuvent en outre, contenir un ou plusieurs actifs cosmétiques ou dermatologiques en vue d'apporter une valence soin au maquillage.

Ces compositions peuvent, par ailleurs, constituer une composition cosmétique ou dermatologique, de protection, de traitement ou de soin du visage, du cou, des mains ou du corps humains et par exemple constituer une crème de soin, un produit solaire ou de bronzage artificiel une pommade ou un onguent dermatologique.

L'invention est illustrée plus en détail dans les exemples suivants.

### Exemple 1 de dispersion de polymère

On prépare une dispersion de polyméthacrylate de méthyle réticulé par du diméthacrylate d'éthylène glycol, dans de l'isododécane, selon la méthode de l'exemple 2 du document EP-A-749 746, en remplaçant L'ISOPAR L par de l'isododécane. On obtient ainsi une dispersion de particules de polyméthacrylate de méthyle stabilisées en surface dans de l'isododécane par un copolymère dibloc séquencé polystyrène/copoly(éhylène-propylène) vendu sous le nom de KRATON G1701 (Shell), ayant un taux de matière sèche de 19,7% en poids et une taille moyenne des particules de 135 nm (polydispersité : 0,05) et une Tg de 100°C. Ce copolymère est non filmifiable à température ambiante.

### Exemple 2 de « fond de teint »

On prépare la composition suivante :
. dispersion de l'exemple 1 83 g
. huile d'amandes d'abricot 8 g
. charges (amidon ou talc) 3,5 g
. oxyde de titane 4 g
. oxyde de fer noir 0,1 g
. oxyde de fer rouge 0,4 g
. oxyde de fer jaune 1 g

Ce fond de teint est préparé de la façon suivante : incorporation des corps gras, et des pigments et charges pour effectuer une bonne dispersion des charges et pigments puis ajout de la dispersion de polymère, à température ambiante (25°C). L'huile non volatile utilisée à un paramètre de solubilité δₕ de 2.

Ce fond de teint fluide, s'étale bien, est non collant, non gras, sans transfert, et camoufle parfaitement bien les taches du visage et des mains.

Un test sensoriel a été effectué avec ce fond de teint par plusieurs expertes ; il a été reconnu confortable et camoufflant parfaitement bien les taches. Son démaquillage par un démaquillant classique (Galateis de Chez Lancôme), sans laisser de traces a été jugé satifaisant.

Ses propriétés de rémanence ont été testées de la façon suivante : dépôt d'une couche mince (d'environ 2mg/cm²) sur un substrat de Vitroskin, trempage dans de l'eau du robinet pendant 20 min. à 28°C. Après, ce trempage dans l'eau chaude, il restait toujours du fond de teint. Ce fond de teint présente bien des propriétés de rémanence à l'eau, permettant son utilisation pour le camouflage permanent des taches des mains.

En outre, ce fond de teint est parfaitement stable dans le temps, puisque placé dans une étuve pendant 2 mois à 45 °C, aucune ségrégation n'est notée. De même au bout de 3 ans à température ambiante (25°C), aucune ségrégation n'est notée.

## Revendications

1. Composition susceptible d'être appliquée sur la peau, se présentant sous forme anhydre, comprenant une phase grasse liquide, des particules de polymère dispersées et stabilisées en surface dans ladite phase grasse liquide et une matière colorante, la phase grasse liquide contenant une phase liquide volatile et une phase liquide non volatile, la phase non volatile ayant un paramètre de solubilité δₕ ≤ 5 (J/cm³)^{1/2} et le rapport, en poids, phase volatile/phase non volatile allant de 2 à 25.

2. Composition selon la revendication 1, dans laquelle le polymère se présente sous forme de particules dispersées et stabilisées en surface par au moins un stabilisant.

3. Composition selon l'une des revendications précédentes, dans laquelle le polymère est choisi parmi les polymères radicalaires, les polycondensats, les polymères d'origine naturelle et leurs mélanges.

4. Composition selon l'une des revendications précédentes, dans laquelle le polymère est choisi parmi les polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

5. Composition selon l'une des revendications précédentes, dans laquelle le polymère est non filmifiable.

6. Composition selon l'une des revendications précédentes, dans laquelle la phase grasse liquide est constitué d'huiles d'origine minérale, animale, végétale ou synthétique, carbonées, hydrocarbonées, fluorées et/ou siliconées, seules ou en mélange, l'une au moins de ces huiles ayant un paramètre de solubilité δₕ ≤ 5 (J/cm³)^{1/2}.

7. Composition selon l'une des revendications précédentes, dans laquelle la phase grasse liquide non volatile est choisie parmi :
- les hydrocarbures linéaires ou ramifiés,
- les huiles hydrocarbonées d'origine animale,
- les huiles hydrocarbonées végétales telles que les triglycérides liquides d'acides gras de 4 à 10 atomes de carbone,
- les esters et les éthers de synthèse notamment d'acides gras comme les huiles de formule R₁CO(O)ₓR₂ dans laquelle R₁ représente le reste d'un acide comportant de 2 à 29 atomes de carbone et R₂ représente une chaîne hydrocarbonée contenant de 3 à 30 atomes de carbone et x vaut de 0 ou 1,
- les esters de polyol,
- les huiles siliconées,
- les polysiloxanes modifiés par des acides gras, des alcools gras ou des polyoxyalkylènes,
- les silicones fluorées,
- les huiles perfluorées,
- leurs mélanges.

8. Composition selon l'une des revendications précédentes, dans laquelle la phase grasse liquide non volatile est choisie parmi :
- les huiles de paraffine, de vaseline et de naphtalène légères, la lanoline,
- le squalène ;
- les huiles de tournesol, d'olive, de bourrache, de maïs, de son de riz, de germes de blé, de soja, de courge, de rosier muscat, de pépins de cassis, de sésame, de noisette, d'abricot, d'arara, de macadamia, d'avocat, de jojoba, de limnanthes alba raffinée,
- l'acétyl-citrate de tributyle, l'érucate d'oléyle, le béhénate d'octyl-2 dodécyle, le citrate de tri-iso-arachidyle, le stéaroyl-stéarate d'isocétyle ou d'octyldodécanyle, l'acétate de n-propyle, le tri-méllitate de tri-décyle, le dodécane di-oléate ou le stéarate de di-iso-cétyle, le propionate d'arachidyle, le dibutyl phtalate, le carbonate de propylène, le pentanoate d'octyldodécyle,
- la vitamine F, l'isostéarate de sorbitane, le triisostéarate de glycérine ou de diglycérine,
- les polydiméthylsiloxanes (PDMS), comportant une chaîne alkyle ou alcoxy en C₃-C₄₀ ou une chaîne phénylée telle que les phényltriméthicones, les polyalkylemethylsiloxanes, éventuellement fluorés comme les polyméthyltrifuoro-propyldiméthylsiloxanes, ou par des groupements fonctionnels tels que des groupements hydroxyle, thiol et/ou amine,
- et leurs mélanges.

9. Composition selon l'une des revendications précédentes, dans laquelle le rapport en poids de phase volatile/phase non volatile est choisi dans la gamme de 5 à 15.

10. Composition selon l'une des revendications précédentes, caractérisée en ce que la phase grasse liquide volatile contient au moins une huile choisie parmi les isoalcanes en C₈-C₁₆ et les esters ramifiés en C₈-C₁₆ et leurs mélanges

11. Composition selon l'une des revendications précédentes, dans laquelle la phase grasse liquide non volatile représente de 1 à 15 % du poids total de la composition.

12. Composition selon l'une des revendications 2 à 11, dans laquelle le stabilisant est choisi parmi les polymères séquencés, les polymères greffés, les polymères statistiques et leurs mélanges.

13. Composition selon la revendication 12, dans laquelle le stabilisant est choisi parmi les polymères siliconés greffés avec une chaîne hydrocarbonée ; les polymères hydrocarbonés greffés avec une chaîne siliconée ; les copolymères greffés ayant un squelette insoluble de type polyacrylique avec des greffons solubles de type acide poly-12(hydroxystéarique) ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères d'acrylates ou de méthacrylates d'alkyle en C₁-C₄, ou d'acrylates ou de méthacrylates d'alkyle en C₈-C₃₀; les copolymères blocs greffés ou séquencés comprenant au moins un bloc résultant de la polymérisation d'un monomère à une ou plusieurs liaisons éthylèniques et au moins un bloc d'un polymère vinylique ; les copolymères blocs greffés ou séquencés comprenant au moins un bloc résultant de la polymérisation d'un monomère à une ou plusieurs liaisons éthylèniques et au moins un bloc d'un polymère acrylique ; les copolymères blocs greffés ou séquencés comprenant au moins un bloc résultant de la polymérisation d'un monomère à une ou plusieurs liaisons éthylèniques et au moins un bloc d'un polyéther.

14. Composition selon la revendication 12 ou 13, caractérisée en ce que le stabilisant est un polymère bloc greffé ou séquencé, comprenant au moins un bloc résultant de la polymérisation de monomère à une ou plusieurs liaisons éthyliniques et au moins un bloc d'un polymère vinylique ou comprenant un bloc polyoxypropyléné et/ou oxyéthyléné et un bloc résultant de la polymérisation d'un siloxane.

15. Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconé, et leurs mélanges.

16. Composition selon l'une des revendications précédentes, caractérisée en ce que la matière colorante comprend des composés pulvérulents choisis parmi les pigments et/ou les nacres et/ou des colorants lipophiles.

17. Composition selon l'une des revendications précédentes caractérisée en ce qu'elle contient, de plus, des charges.

18. Composition selon la revendication 16, caractérisée en ce que les composés pulvérulents représentent jusqu'à 30 % du poids total de la composition.

19. Composition selon l'une des revendications précédentes, caractérisée en ce que le polymère représente (en matière sèche) jusqu'à 30 % du poids total de la composition.

20. Composition selon l'une des revendications précédentes, se présentant sous forme d'un gel anhydre.

21. Composition selon l'une des revendications précédentes, se présentant sous la forme d'un produit de soin et/ou de maquillage de la peau.

22. Composition selon l'une des revendications précédentes, se présentant sous la forme d'un fond de teint, d'un fard à joues ou à paupières, d'une crème de soin ou de protection de la peau, d'un produit de soin ou de maquillage du corps, d'une crème solaire ou de bronzage artificiel de la peau.

23. Composition selon l'une des revendications précédentes, caractérisé en ce qu'elle contient, en outre, au moins un actif cosmétique ou dermatologique.

24. Utilisation dans une composition cosmétique ou pour la fabrication d'une composition physiologiquement acceptable de particules de polymère dispersées et stabilisées en surface dans une phase grasse liquide, pour diminuer, voire supprimer les taches, les dischromies, les défauts de pigmentation, la couperose et/ou les irrigations sanguines diffuses apparaissant à travers la peau, ladite phase grasse liquide contenant une phase liquide volatile et une phase liquide non volatile, la phase non volatile ayant un paramètre de solubilité δh ≤ 5 (J/cm³)^{½} et le rapport, en poids, phase volatile/phase non volatile allant de 2 à 25, ladite composition contenant une matière colorante.

25. Procédé non thérapeutique et en particulier cosmétique de camouflage notamment rémanent des taches, des défauts de pigmentation de la peau, des dischromies, de la couperose, des irrigations sanguines diffuses apparaissant à travers la peau et des télangiectasies, consistant à introduire dans une composition contenant une matière colorante, une phase grasse liquide contenant une phase liquide volatile, une phase liquide non volatile et des particules de polymère dispersées et stabilisées en surface dans ladite phase grasse liquide, la phase non volatile ayant un paramètre de solubilité δₕ ≤ 5 (J/cm³)^{1/2} et le rapport, en poids, phase volatile/phase non volatile allant de 2 à 25.

## Patentansprüche

1. Zusammensetzung, die auf die Haut aufgetragen werden kann, die in wasserfreier Form vorliegt und die eine flüssige Fettphase, Polymerpartikel, die in der flüssigen Fettphase an der Oberfläche stabilisiert und dispergiert sind, und ein Färbemittel enthält, wobei die flüssige Fettphase eine flüchtige, flüssige Phase und eine nicht flüchtige, flüssige Phase umfaßt, wobei die nicht flüchtige Phase einen Solubilitätsparameter δₕ ≤ 5 (J/cm³)^{1/2} aufweist und wobei das Gewichtsverhältnis von flüchtiger Phase und nicht flüchtiger Phase im Bereich von 2 bis 25 liegt.

2. Zusammensetzung nach Anspruch 1, wobei das Polymer in Form von Partikel vorliegt, die durch mindestens ein Stabilisierungsmittel an der Oberfläche stabilisiert und dispergiert sind.

3. Zusammensetzung nach einem der vorhergehenden Ansprüche, wobei das Polymer unter den durch radikalische Polymerisation hergestellten Polymeren, Polykondensaten, Polymeren natürlicher Herkunft und deren Gemischen ausgewählt ist.

4. Zusammensetzung nach einem der vorhergehenden Ansprüche, wobei das Polymer unter den Polyurethanen, Polyurethan-Acrylpolymeren, Polyharnstoffen, Polyharnstoff-Polyurethanen, Polyester-Polyurethanen, Polyether-Polyurethanen, Polyestern, Polyesteramiden, Polyestern mit Fettkette und Alkydharzen; Acryl- und/oder Vinylpolymeren oder Acryl- und/oder Vinylcopolymeren; Acryl-Silicon-Copolymeren; Polyacrylamiden; Siliconpolymeren, fluorierten Polymeren und deren Gemischen ausgewählt ist.

5. Zusammensetzung nach einem der vorhergehenden Ansprüche, wobei das Polymer nicht filmbildend ist.

6. Zusammensetzung nach einem der vorhergehenden Ansprüche, wobei die flüssige Fettphase aus Ölen mineralischen, tierischen, pflanzlichen oder synthetischen Ursprungs, Ölen auf Kohlenstoffbasis, Kohlenwasserstoffölen, fluorierten Ölen und/oder Siliconölen oder Gemischen dieser Öle besteht, wobei mindestens ein Öl einen Solubilitätsparameter δₕ ≤ 5 (J/cm³)^{1/2} aufweist.

7. Zusammensetzung nach einem der vorhergehenden Ansprüche, wobei die nicht flüchtige, flüssige Fettphase ausgewählt ist unter:
- geradkettigen oder verzweigten Kohlenwasserstoffen,
- Kohlenwasserstoffölen tierischer Herkunft,
- pflanzlichen Kohlenwasserstoffölen, wie flüssigen Triglyceriden von Fettsäuren mit 4 bis 10 Kohlenstoffatomen,
- synthetischen Estern oder Ethern, insbesondere von Fettsäuren, wie Öle der Formel R₁CO(O)ₓR₂, worin R₁ den Rest einer Säure mit 2 bis 29 Kohlenstoffatomen und R₂ eine Kohlenwasserstoffkette mit 3 bis 30 Kohlenstoffatomen bedeutet und x 0 oder 1 ist,
- Polyolestern,
- Siliconölen,
- mit Fettsäuren, Fettalkoholen oder Polyoxyalkylenen modifizierten Polysiloxanen,
- fluorierten Siliconen,
- perfluorierten Ölen; und
- deren Gemischen.

8. Zusammensetzung nach einem der vorhergehenden Ansprüche, wobei die nicht flüchtige, flüssige Fettphase ausgewählt ist unter:
- Paraffinöl, Vaselineöl und Naphthalinöl, die niedrig sieden, und Lanolin,
- Squalen,
- Sonnenblumenöl, Olivenöl, Borretschöl, Maisöl, Reiskleieöl, Weizenkeimöl, Sojaöl, Kürbiskernöl, Öl aus Rosa mosqueta, Johanniskernöl, Sesamöl, Haselnußöl, Aprikosenkernöl, Araraöl, Macadamiaöl, Avocadoöl, Jojobaöl und aufbereitetem Öl aus Limnanthes alba,
- Tributylacetylcitrat, Oleylerucat, 2-Octyldodecylbeheneat, Triisoarachidylcitrat, Isocetylstearoylstearat, Octyldodecanylstearoylstearat, n-Propylacetat, Tridecyltrimellitat, Dodecandioleat, Düsocetylstearat, Arachidylpropionat, Dibutylphthalat, Propylencarbonat und Octyldodecylpentanoat,
- Vitamin F, Sorbitanisostearat, Glycerintriisostearat oder Diglycerintrüsostearat,
- Polydimethylsiloxane (PDMS), die eine Alkyl- oder Alkoxygruppe mit 3 bis 40 Kohlenstoffatomen oder Phenylgruppen enthalten, beispielsweise Phenyltrimethicone, Polyalkylmethylsiloxane, die gegebenenfalls fluoriert sind, beispielsweise Polymethyltrifluorpropyldimethylsiloxane, oder mit funktionellen Gruppen substituiert sind, wie Hydroxy-, Thiol- und/oder Aminogruppen, und
- deren Gemischen.

9. Zusammensetzung nach einem der vorhergehenden Ansprüche, wobei das Gewichtsverhältnis von flüchtiger Phase und nicht flüchtiger Phase im Bereich von 5 bis 15 liegt.

10. Zusammensetzung nach einem der vorhergehenden Ansprüche, dadurch gekennzeichnet, daß die flüchtige, flüssige Fettphase mindestens ein Öl enthält, das unter den C₈₋₁₆-Isoalkanen und den verzweigten C₈₋₁₆-Estern und deren Gemischen ausgewählt ist.

11. Zusammensetzung nach einem der vorhergehenden Ansprüche, wobei die nicht flüchtige, flüssige Fettphase 1 bis 15 Gew.-% des Gesaxntgewichts der Zusammensetzung ausmacht.

12. Zusammensetzung nach einem der Ansprüche 2 bis 11, wobei das Stabilisierungsmittel unter den Blockpolymeren, Pfropfpolymeren, statistischen Polymeren und deren Gemischen ausgewählt ist.

13. Zusammensetzung nach Anspruch 12, wobei das Stabilisierungsmittel ausgewählt ist unter: den mit einer Kohlenwasserstoffkette gepfropften Siliconpolymeren; den mit einer Siliconkette gepfropften Kohlenwasserstoffpolymeren; den gepfropften Copolymeren, die ein unlösliches Grundgerüst vom Polyacryltyp mit löslichen Pfropfzweigen vom Poly-12-hydroxystearinsäuretyp aufweisen; den gepfropften oder sequentiellen Blockcopolymeren, die mindestens einen Block vom Polyorganosiloxantyp und mindestens einen Block eines durch radikalische Polymerisation hergestellten Polymers enthalten; den gepfropften oder sequentiellen Blockcopolymeren, die mindestens einen Block vom Polyorganosiloxantyp und mindestens einen Polyetherblock aufweisen; den Copolymeren von Acrylaten oder Methacrylaten von C₁₋₄-Alkoholen und Acrylaten oder Methacrylaten von C₈₋₃₀-Alkoholen; den gepfropften oder sequentiellen Blockcopolymeren, die mindestens einen Block, der bei der Polymerisation von Monomeren mit einer oder mehreren ethylenischen Doppelbindungen gebildet wird, und mindestens einen Vinylpolymerblock enthalten; den gepfropften oder sequentiellen Blockcopolymeren, die mindestens einen Block, der bei der Polymerisation von Monomeren mit einer oder mehreren ethylenischen Doppelbindungen gebildet wird, und mindestens einen Acrylpolymerblock enthalten; und den gepfropften oder sequentiellen Blockcopolymeren, die mindestens einen Block, der bei der Polymerisation von Monomeren mit einer oder mehreren ethylenischen Doppelbindungen gebildet wird, und mindestens einen Polyetherblock enthalten.

14. Zusammensetzung nach Anspruch 12 oder 13, dadurch gekennzeichnet, daß das Stabilisierungsmittel ein gepfropftes oder sequentielles Blockcopolymer ist, das mindestens einen Block, der bei der Polymerisation von Monomeren mit einer oder mehreren ethylenischen Doppelbindungen gebildet wird, und mindestens einen Vinylpolymerblock oder einen Polypropylenoxid- und/oder Polyethylenoxid-Block und einen bei der Polymerisation eines Siloxans resultierenden Block enthält.

15. Zusammensetzung nach einem der vorhergehenden Ansprüche, die ferner mindestens eine zusätzliche Fettphase enthält, die unter den Wachsen, Gummen/Gummis und/oder pastösen Fettsubstanzen pflanzlichen, tierischen, mineralischen oder synthetischen Ursprungs oder auf Siliconbasis und deren Gemischen ausgewählt ist,

16. Zusammensetzung nach einem der vorhergehenden Ansprüche, dadurch gekennzeichnet, daß das Färbemittel pulverförmige Verbindungen umfaßt, die unter den Pigmenten und/oder Perlglanzpigmenten und/oder lipophilen Farbstoffen ausgewählt sind.

17. Zusammensetzung nach einem der vorhergehenden Ansprüche, dadurch gekennzeichnet, daß sie außerdem Füllstoffe enthält.

18. Zusammensetzung nach Anspruch 16, dadurch gekennzeichnet, daß die pulverförmigen Verbindungen bis zu 30 % des Gesamtgewichts der Zusammensetzung ausmachen.

19. Zusammensetzung nach einem der vorhergehenden Ansprüche, dadurch gekennzeichnet, daß das Polymer (als Trockensubstanz) bis zu 30 % des Gesamtgewichts der Zusammensetzung ausmacht.

20. Zusammensetzung nach einem der vorhergehenden Ansprüche, die in Form eines wasserfreien Gels vorliegt.

21. Zusammensetzung nach einem der vorhergehenden Ansprüche, die in Form eines Produktes zur Pflege und/oder zum Schminken der Haut vorliegt.

22. Zusammensetzung nach einem der vorhergehenden Ansprüche, die als Make-up, Wangenrouge, Lidschatten, Creme zur Pflege oder zum Schutz der Haut, Produkt zur Pflege oder zum Schminken des Körpers, Sonnenschutzcreme oder Creme zur künstlichen Bräunung der Haut vorliegt.

23. Zusammensetzung nach einem der vorhergehenden Ansprüche, dadurch gekennzeichnet, daß sie ferner mindestens einen kosmetischen oder dermatologischen Wirkstoff enthält.

24. Verwendung von Partikeln eines Polymers, die in einer flüssigen Fettphase an der Oberfläche stabilisiert und dispergiert sind, in einer kosmetischen Zusammensetzung oder zur Herstellung einer physiologisch akzeptablen Zusammensetzung, um Flecken, Dyschromien, Pigmentierungsstörungen, Acne rosacea und/oder diffuse Irrigationen von Blut, die durch die Haut durchscheinen, zu vermindern oder zu beseitigen, wobei die flüssige Fettphase eine flüchtige, flüssige Phase und eine nicht flüchtige, flüssige Phase umfaßt, wobei die nicht flüchtige Phase einen Solubilitätsparameter δₕ ≤ 5 (J/cm³)^{1/2} aufweist, wobei das Gewichtsverhältnis von flüchtiger Phase und nicht flüchtiger Phase im Bereich von 2 bis 25 liegt und wobei die Zusammensetzung ein Färbemittel enthält.

25. Nicht therapeutisches und insbesondere kosmetisches Verfahren zum Abdecken und insbesondere dauerhaften Abdecken von Flekken, Figmentierungsstörungen der Haut, Dyschromien, Acne rosacea, diffusen Irrigationen von Blut, die durch die Haut durchscheinen, und Teleangiektasien, das darin besteht, eine Zusammensetzung, die ein Färbemittel, eine flüssige Fettphase, die eine flüchtige, flüssige Phase und eine nicht flüchtige, flüssige Phase umfaßt, und Polymerpartikel enthält, die in der flüssigen Fettphase an der Oberfläche stabilisiert und dispergiert sind, wobei die nicht flüchtige Phase einen Solubilitätsparameter δₕ ≤ 5 (J/cm³)^{1/2} aufweist und das Gewichtsverhältnis von flüchtiger Phase und nicht flüchtiger Phase im Bereich von 2 bis 25 liegt.

## Claims

1. Composition which can be applied to the skin and which is in anhydrous form, comprising a liquid' fatty phase, polymer particles that are dispersed and surface-stabilized in the said liquid fatty phase and a dyestuff, the liquid fatty phase containing a volatile liquid phase and a non-volatile liquid phase, the non-volatile phase having a solubility parameter δₕ ≤5 (J/cm³)^{½} and the volatile phase/non-volatile phase weight ratio ranging from 2 to 25.

2. Composition according to Claim 1, in which the polymer is in the form of particles that are dispersed and surface-stabilized by at least one stabilizer.

3. Composition according to either of the preceding claims, in which the polymer is chosen from radical-mediated polymers, polycondensates, polymers of natural origin and mixtures thereof.

4. Composition according to one of the preceding claims, in which the polymer is chosen from polyurethanes, polyurethane-acrylics, polyureas, polyurea-polyurethanes, polyester-polyurethanes, polyether-polyurethanes, polyesters, polyesteramides, fatty-chain polyesters, alkyds; acrylic and/or vinyl polymers or copolymers; acrylic-silicone copolymers; polyacrylamides; silicone polymers, fluoro polymers and mixtures thereof.

5. Composition according to one of the preceding claims, in which the polymer is non-film-forming.

6. Composition according to one of the preceding claims, in which the liquid fatty phase consists of oils of mineral, animal, plant or synthetic, carbon-based, hydrocarbon-based, fluoro and/or silicone origin, alone or as a mixture, at least one of these oils having a solubility parameter δₕ ≤ 5 (J/cm³)^{½}.

7. Composition according to one of the preceding claims, in which the non-volatile liquid fatty phase is chosen from:
- linear or branched hydrocarbons,
- hydrocarbon-based oils of animal origin,
- hydrocarbon-based plant oils such as liquid triglycerides of fatty acids containing from 4 to 10 carbon atoms,
- synthetic esters and ethers, in particular of fatty acids, such as oils of formula R₁CO(O)ₓR₂ in which R₁ represents an acid residue comprising from 2 to 29 carbon atoms and R₂ represents a hydrocarbon-based chain containing from 3 to 30 carbon atoms and x is 0 or 1,
- polyol esters,
- silicone oils,
- polysiloxanes modified with fatty acids, fatty alcohols or polyoxyalkylenes,
- fluorosilicones,
- perfluoro oils,
- mixtures thereof.

8. Composition according to one of the preceding claims, in which the non-volatile liquid fatty phase is chosen from:
- liquid paraffin, liquid petroleum jelly and light naphthalene oils, and lanolin,
- squalene,
- sunflower oil, olive oil, borage oil, corn oil, rice bran oil, wheatgerm oil, soybean oil, marrow oil, musk rose oil, blackcurrant pip oil, sesame oil, hazelnut oil, apricot oil, arara oil, macadamia oil, avocado oil, jojoba oil or refined oil of Limnanthes alba,
- tributyl acetyl citrate, oleyl erucate, 2-octyldodecyl behenate, triisoarachidyl citrate, isocetyl or octyldodecyl stearoylstearate, n-propyl acetate, tridecyl trimellitate, diisocetyl dodecane di-oleate or stearate, arachidyl propionate, dibutyl phthalate, propylene carbonate, octyldodecyl pentanoate,
- vitamin F, sorbitan isostearate, glyceryl or diglyceryl triisostearate,
- polydimethylsiloxanes (PDMSs) comprising a C₃-C₄₀ alkyl or alkoxy chain or a phenyl chain, such as phenyltrimethicones, optionally fluorinated polyalkylmethylsiloxanes, such as polymethyltrifluoropropyldimethylsiloxanes, or with functional groups such as hydroxyl, thiol and/or amine groups,
- and mixtures thereof.

9. Composition according to one of the preceding claims, in which the volatile phase/nonvolatile phase weight ratio is chosen in the range from 5 to 15.

10. Composition according to one of the preceding claims, characterized in that the volatile liquid fatty phase contains at least one oil chosen from C₈-C₁₆ isoalkanes and C₈-C₁₆ branched esters and mixtures thereof.

11. Composition according to one of the preceding claims, in which the non-volatile liquid fatty phase represents from 1 to 15% of the total weight of the composition.

12. Composition according to one of Claims 2 to 11, in which the stabilizer is chosen from block polymers, grafted polymers, random polymers and mixtures thereof.

13. Composition according to Claim 12, in which the stabilizer is chosen from silicone polymers grafted with a hydrocarbon-based chain; hydrocarbon-based polymers grafted with a silicone chain; copolymers grafted with an insoluble skeleton of polyacrylic type with soluble grafts of poly-12-(hydroxystearic) acid type; block or grafted block copolymers comprising at least one block of polyorganosiloxane type and at least one block of a radical-mediated polymer; block or grafted block copolymers comprising at least one block of polyorganosiloxane type and at least one polyether; copolymers of C₁-C₄ alkyl acrylates or methacrylates, or of C₈-C₃₀ alkyl acrylates or methacrylates; block or grafted block copolymers comprising at least one block resulting from the polymerization of a monomer containing one or more ethylenic bonds and at least one block of a vinyl polymer; block or grafted block copolymers comprising at least one block resulting from the polymerization of a monomer containing one or more ethylenic bonds and at least one block of an acrylic polymer; block or grafted block copolymers comprising at least one block resulting from the polymerization of a monomer containing one or more ethylenic bonds and at least one block of a polyether.

14. Composition according to Claim 12 or 13, characterized in that the stabilizer is a block or grafted block polymer, comprising at least one block resulting from the polymerization of a monomer containing one or more ethylenic bonds and at least one block of a vinyl polymer, or comprising a polyoxypropylenated and/or oxyethylenated block and a block resulting from the polymerization of a siloxane.

15. Composition according to one of the preceding claims, also comprising at least one additional fatty phase chosen from waxes, gums and/or pasty fatty substances of plant, animal, mineral, synthetic or silicone origin, and mixtures thereof.

16. Composition according to one of the preceding claims, characterized in that the dyestuff comprises pulverulent compounds chosen from pigments and/or pearlescent agents and/or lipophilic dyes.

17. Composition according to one of the preceding claims, characterized in that it also contains fillers.

18. Composition according to Claim 16, characterized in that the pulverulent compounds represent up to 30% of the total weight of the composition.

19. Composition according to one of the preceding claims, characterized in that the polymer represents (as solids) up to 30% of the total weight of the composition.

20. Composition according to one of the preceding claims, in the form of an anhydrous gel.

21. Composition according to one of the preceding claims, in the form of a care product and/or make-up product for the skin.

22. Composition according to one of the preceding claims, in the form of a foundation, a face powder, an eyeshadow, a care cream or protective cream for the skin, a care product or make-up product for the body, an antisun cream or a cream for artificially tanning the skin.

23. Composition according to one of the preceding claims, characterized in that it also contains at least one cosmetic or dermatological active agent.

24. Use, in a cosmetic composition or for the manufacture of a physiologically acceptable composition, of polymer particles that are dispersed and surface-stabilized in a liquid fatty phase, to reduce or even eliminate marks, dyschromia, pigmentation defects, couperose and/or diffuse blood irrigations appearing through the skin, the said liquid fatty phase containing a volatile liquid phase and a non-volatile liquid phase, the non-volatile phase having a solubility parameter δₕ ≤ 5 (J/cm³)^{½} and the volatile phase/non-volatile phase weight ratio ranging from 2 to 25, the said composition containing a dyestuff.

25. Non-therapeutic process, and in particular cosmetic process, for the camouflaging, in particular the remanent camouflaging of marks, skin pigmentation defects, dyschromia, couperose, diffuse blood irrigations appearing through the skin and telangiectasias, which consists in introducing, into a composition containing a dyestuff, a liquid fatty phase containing a volatile liquid phase, a non-volatile liquid phase and polymer particles that are dispersed and surface-stabilized in the said .liquid fatty phase, the non-volatile phase having a solubility parameter δₕ ≤ 5 (J/cm³)^{½} and the volatile phase/non-volatile phase weight ratio ranging from 2 to 25.
